# EUROPÄISCHE PATENTSCHRIFT

(11) **EP 1 291 081 B1**
(45) Veröffentlichungstag und Bekanntmachung des Hinweises auf die Patenterteilung: **31.12.2008**
(21) Anmeldenummer: 02019883.4
(22) Anmeldetag: 10.09.2002
(51) Int. Cl.: B01J 23/42, B01J 23/44, C07D 207/32, B01J 23/63, C07D 213/06

(54) **Verfahren zur Herstellung von Pyrrolen**
Process for the preparation of pyrroles
Procédé de production de pyrroles

(30) Priorität: 11.09.2001 DE 10144631
(43) Veröffentlichungstag der Anmeldung: 12.03.2003
(73) Patentinhaber: BASF SE, 67056 Ludwigshafen (DE)
(72) Erfinder: Schäfer, Martin, Dr., 67269 Grünstadt (DE); Böttcher, Arnd, Dr., 67227 Frankenthal (DE); Kramer, Andreas, Dr., 67098 Bad Dürkheim (DE); Höhn, Arthur, Dr., 67281 Kirchheim (DE); Kaczmarek, Reinhard, Dr., 67454 Hassloch (DE); Henkes, Erhard, Dr., 64683 Einhausen (DE)
(74) Vertreter: Isenbruck, Günter

(56) Entgegenhaltungen:
- EP-A- 0 608 688
- EP-A- 1 046 639
- WO-A-89/08103
- WO-A-94/22824

## Beschreibung

Die vorliegende Erfindung betrifft ein Verfahren zur Herstellung von Pyrrolen aus Pyrrolidinen durch Dehydrieren an Edelmetallkatalysatoren.

Es ist bekannt, dass man Pyrrolidin und Piperidin an Palladium und/oder Platin enthaltenden Trägerkatalysatoren zu Pyrrol bzw. Pyridin dehydrieren kann.

In der US-A 3 522 269 wird die Dehydrierung von Pyrrolidin zu Pyrrol an Pd-Katalysatoren bei relativ hohen Temperaturen, bevorzugt 400 - 450°C, beschrieben.

In der GB-A 1 393 086 wird die Dehydrierung von Piperidin zu Pyridin mittels Katalysatoren aus Palladium auf SiO₂ als Träger beschrieben.

In den EP-A 67 360 und 155 649 werden Pd-Katalysatoren zur Dehydrierung von Pyrrolidinen beschrieben, die bei niedrigeren Temperaturen von 160 bis 400 °C wirksam sind.

In der EP-A-0608688 wird die Dehydrierung von Pyrrolidin beschrieben, wobei ein wasserhaltigen Rohpyrrol entsteht.

Bei der Dehydrierung von Pyrrolidin zu Pyrrol werden in gewissem Umfang Nebenprodukte wie Butylamin, N-Butylpyrrol und Butyronitril gebildet. Von diesen ist Butyronitril, das im allgemeinen in Mengen von 0,3 bis 3 Gew.-% gebildet wird, besonders störend. Wegen der stark ähnlichen Siedepunkte von Pyrrol und Butyronitril kann Butyronitril nur mit erheblichem Aufwand von Pyrrol destillativ abgetrennt werden. Dazu sind Kolonnen mit einer hohen Trennstufenzahl erforderlich, und es muss mit einem hohen Rücklaufverhältnis gearbeitet werden. Die destillative Trennung ist mit einem hohen Verlust an Wertprodukt verbunden. Der Einsatz der Pyrrole bei der Herstellung von pharmazeutischen Wirkstoffen oder in elektronischen Bauteilen erfordert jedoch einen sehr hohen Reinheitsgrad der Pyrrole.

Aufgabe der Erfindung ist, ein verbessertes Verfahren zur Herstellung von Pyrrolen bereitzustellen, bei dem Nebenprodukte in geringerem Ausmaß gebildet werden.

Gelöst wird die Aufgabe durch ein Verfahren zum Herstellen von Pyrrolen der allgemeinen Formel (I) 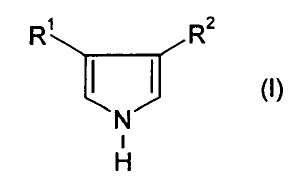 worin
- R¹ und R²: unabhängig voneinander Wasserstoff oder einen aliphatischen Rest mit 1 bis 6 C-Atomen bedeuten,
durch Dehydrieren von Pyrrolidinen der allgemeinen Formel (II) 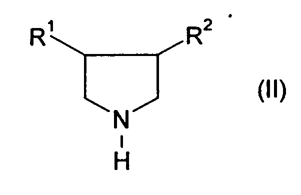 worin R¹ und R² die oben angegebene Bedeutung haben,
in Gegenwart eines geträgerten Edelmetallkatalysators, dadurch gekennzeichnet, dass man die Dehydrierung in Gegenwart von 1 bis 50 Gew.-% Wasser, bezogen auf Pyrrolidin und Wasser, durchführt, wobei wasser bereits dem Einsatzstrom der Dehydrierung beigemischt ist.

Es wurde gefunden, dass durch die Gegenwart von Wasser während der Dehydrierung von (substituiertem) Pyrrolidin die Bildung von (substituiertem) Butyronitril stark zurückgedrängt wird. Die Menge an Butyronitril im Rohaustrag der Dehydrierung von Pyrrolidin beträgt im allgemeinen < 0,2 Gew.-%, vorzugsweise < 0,1 Gew.-%. Dadurch wird die nachfolgende Reindestillation von Pyrrol erheblich vereinfacht. Es kommt nicht zu den hohen Verlusten an Wertprodukt, wodurch die Herstellung von Pyrrol insgesamt wirtschaftlicher wird.

Als Pyrrolidine (I) kommen in Betracht: unsubstituiertes oder in 3- und/oder 4-Position mit einem aliphatischen Rest mit 1 bis 6 C-Atomen substituiertes Pyrrolidin. Die aliphatischen Reste können mit unter den Reaktionsbedingungen inerten Gruppen substituiert sein. Bevorzugte aliphatische Reste sind Methyl, Ethyl, Propyl, Isopropyl, Butyl, Isobutyl, sek.-Butyl, tert.-Butyl sowie isomere Pentyl- und Hexylreste. Bevorzugte Ausgangsverbindung der erfindungsgemäßen Dehydrierung ist unsubstituiertes Pyrrolidin.

Die Dehydrierung wird in Gegenwart von 1 bis 50 Gew.-%, vorzugsweise 5 bis 50 Gew.-%, besonders bevorzugt 5 bis 25 Gew.-% Wasser durchgeführt. Die %-Angaben beziehen sich dabei auf die Summe aus Pyrrolidin und Wasser, die 100 Gew.-% ergibt. Das Wasser wird bereits dem Pyrrolidin- Einsatzstrom beigemischt.

Die erfindungsgemäße katalytische Dehydrierung kann in flüssiger Phase oder in der Gasphase durchgeführt werden.

Die erfindungsgemäße katalytische Dehydrierung kann in der Flüssigphase durchgeführt werden. Dazu werden Pyrrolidin und der edelmetallhaltige Trägerkatalysator, gegebenenfalls in einem Lösungsmittel, beispielsweise 1 bis 10 h bei der Reaktionstemperatur gehalten. Aus dem erhaltenen Produktgemisch wird der Katalysator durch Filtration abgetrennt und Pyrrol durch fraktionierte Destillation gewonnen. Bevorzugte Lösungsmittel sind Toluol, Xylole, Diethylbenzol, Ethanol, n-Butanol, Methylglykol, Ethylenglykol und 1,2-Dimethoxyethan. Die Dehydrierung in flüssiger Phase wird üblicherweise in Gegenwart geringer Mengen Wasserstoff durchgeführt, um den Katalysator zu aktivieren.

Vorzugsweise wird die erfindungsgemäße Dehydrierung kontinuierlich in der Gasphase durchgeführt, wobei der Katalysator in einem Festbett oder als Wirbelbett angeordnet sein kann.

Wird die erfindungsgemäße Dehydrierung in der Gasphase durchgeführt, so liegen das Pyrrolidin/Wasserdampf- Gemisch vorzugsweise in einem Trägergasstrom aus Stickstoff und/oder Wasserstoff vor. Das Volumenverhältnis Trägergas : Pyrrolidin beträgt in der Regel 1:1 bis 100:1, bevorzugt 2:1 bis 50:1, besonders bevorzugt 3:1 bis 40:1. Das Volumenverhältnis von Stickstoff zu Wasserstoff kann 0,01:1 bis 100:1, bevorzugt 0,1:1 bis 10:1, besonders bevorzugt 0,5:1 bis 5:1 betragen.

Die erfindungsgemäße Dehydrierung kann in Gegenwart aller für diese Dehydrierungsreaktion üblichen edelmetallhaltigen Trägerkatalysatoren durchgeführt werden. Dabei wird im allgemeinen bei Temperaturen von 150 bis 400 °C und Drücken von 0,01 bis 50 bar gearbeitet.

Die erfindungsgemäße Dehydrierung kann beispielsweise in Gegenwart der in EP-A 0 067 360 beschriebenen Palladiumträgerkatalysatoren, die basische Verbindungen und/oder Elemente der Gruppe 11, Gruppe 12, Gruppe 7, Cobalt und/oder Nickel enthalten, durchgeführt werden. Träger sind beispielsweise Aluminiumoxid, Siliciumdioxid, Aluminiumsilikat, Magnesiumsilikat oder Spinelle des Aluminiums, Chroms oder Eisens. Als aktivierende Zusatzelemente der Gruppen 7, 11 und 12 bevorzugt sind Mangan, Zink und Silber. Bevorzugte basische Verbindungen sind die Oxide, Hydroxide oder Carbonate der Alkalimetalle, vorzugsweise des Lithiums, der Erdalkalimetalle, vorzugsweise des Magnesiums und Calciums, und der Lanthaniden, vorzugsweise des Cers, Praseodyms und Neodyms.

Bevorzugt sind jedoch edelmetallhaltige Katalysatoren, die 30 bis 100 Gew.-%
a) Palladium auf einem Oxid einer Seltenen Erde oder einem Oxid eines Elements der Gruppe 4 oder
b) eines Platin/Palladium-Gemischs auf Aluminiumoxid, einem Oxid einer Seltenen Erde oder einem Oxid eines Elements der Gruppe 4
und 0 bis 70 Gew.-% Alkali- oder Erdalkalimetalloxid enthalten. In Gegenwart dieser Katalysatoren wird die erfindungsgemäße Dehydrierung im allgemeinen bei Temperaturen im Bereich von 150 bis 300°C, bevorzugt 170 bis 270°C, besonders bevorzugt 180 bis 250°C und Drücken von 0,01 bis 50 bar, bevorzugt 0,1 bis 5 bar, besonders bevorzugt von 1 bis 1,5 bar durchgeführt.

Als Seltene Erden im Sinn dieser Beschreibung eigenen sich die Elemente der Lanthaniden- und der Actinidengruppe des Periodensystems, wie Lanthan, Cer, Neodym, Samarium, Gadolinium, Ytterbium, Actinium, Thorium, Uran und Neptunium, bevorzugt sind Cer, Praseodym, Neodym, Samarium, Europium, Terbium, Ytterbium, Thorium und Protactinium, besonders bevorzugt sind Cer, Praseodym, Neodym und Thorium.

Als Metalle der Gruppe 4 eignen sich Titan, Zirkonium und Hafnium, bevorzugt sind Titan und Zirkonium, besonders bevorzugt ist Zirkonium.

Als Alkali- oder Erdalkalimetalle eignen sich Lithium, Natrium, Kalium, Caesium, Beryllium, Magnesium, Calcium, Strontium und Barium, bevorzugt sind Natrium, Kalium, Magnesium, Calcium und Barium.

Die aktiven Bestandteile des Katalysators (Edelmetalle) befinden sich, wenn es sich um reines Palladium handelt, vorzugsweise auf Oxiden der Lanthaniden oder Actiniden oder auf Oxiden von Elementen der Gruppe 4, und, wenn es sich um Platin/Palladium-Gemische handelt, vorzugsweise auf im wesentlichen aus Aluminiumoxid aufgebauten Trägern, auf Oxiden der seltenen Erden oder auf Oxiden der Gruppe 4.

Die Katalysatoren können, soweit vorhanden, durch gemeinsames Kneten der Zusätze (d.h. der Alkali- oder Erdalkalimetalloxide) mit dem Trägermaterial, thermische Nachbehandlung (Tempern) bei 400 bis 900°C und Tränken mit einer ein Salz des Edelmetalls enthaltenden Lösung oder durch Tränken des Trägers mit einer Lösung der Zusätze und des Edelmetalls, z.B. in Form von Lösungen ihrer Nitrate, Chloride, Formiate, Oxalate oder Ammoniakate und darauf folgendes Tempern bei 400 bis 900°C, hergestellt werden. Wenn eine Spinellbildung bewirkt werden soll, muss nach dem Kneten oder dem Tränken des Aluminiumoxids mit dem Oxid bzw. der Lösung der Zusatzkomponente eine Temperatur von 900 bis 1 300°C erreicht werden (siehe Ullmanns Encyklopädie der technischen Chemie, 3. Auflage (1955) Band 6, Seiten 242 bis 244).

Der Edelmetallgehalt der Katalysatoren, bezogen auf das Trägermaterial, liegt in der Regel bei 0,0001 bis 25 Gew.-%, bevorzugt 0,001 bis 20 Gew.-%, besonders bevorzugt 0,05 bis 15 Gew.-%. Man kann die Katalysatoren beispielsweise in Form von Formkörpern, z.B. Strängen, Tabletten oder Ringen, oder als Pulver je nach dem vorgesehenen Verwendungszweck einsetzen.

Die bevorzugt eingesetzten Katalysatoren haben den Vorteil, dass sie langsamer deaktiviert werden und dass deshalb durch ihren Einsatz bei der Dehydrierung im technischen Betrieb deutlich weniger Unterbrechungen oder Stillstandszeiten zum Katalysatorwechsel erforderlich sind. Sie weisen eine hohe Anfangsselektivität für die Dehydrierung zum aromatischen Heterocyclus auf, so dass ihre Selektivität auch über die gesamte Laufzeit mit einer Katalysatorcharge sehr gut ist. Die Bildung von Pyrrolinen als Nebenprodukte kann dabei weitgehend unterdrückt werden.

Die Reaktion wird bevorzugt in einem Rohrreaktor durchgeführt, da es darauf ankommt, eine Rückvermischung vom Ausgang der Anlage, d.h. dem Ende der letzten Reaktionszone, zum Eingang, d.h. dem Beginn der ersten Reaktionszone, zu vermeiden, was sich in Rohrreaktoren und der sich darin ausbildenden Pfropfenströmung am leichtesten erreichen lässt.

Aus dem erhaltenen Produktgemisch wird Wasser durch Phasentrennung, beispielsweise in einem Mixer-Settler-Apparat, abgetrennt. Anschließend werden Pyrrol, das noch unumgesetztes Pyrrolidin sowie Nebenprodukte enthalten kann, einer Reindestillation unterworfen.

Die nach dem erfindungsgemäßen Verfahren hergestellten Verbindungen können z.B. zur Herstellung von pharmazeutischen Wirkstoffen eingesetzt werden. Die erfindungsgemäß hergestellten Pyrrole können insbesondere in Elektrolytkondensatoren eingesetzt werden. Pyrrole stellen wertvolle Zwischenprodukte für die organische Synthese dar.

Die Erfindung wird durch die nachstehenden Beispiele näher erläutert.

### Beispiele

Die Dehydrierungen wurden in einem elektrisch beheizten Gasphasen-Laborreaktor durchgeführt, der mit einem geträgerten Platin/Palladium-Katalysator (0,5 Gew.-% Pt / 0,5 Gew.-% Pd auf ZrO₂) gefüllt war. Der Reaktor hatte einen Durchmesser von 30 mm und eine Länge von 1000 mm, die Länge des Katalysatorbetts betrug 350 mm. Der Pyrrolidin-Einsatzstrom wurde vor der Dehydrierung mit der in der Tabelle angegebenen Menge Wasser versetzt. Die Reaktion wurde bei Normaldruck und bei 260°C in einem Gasstrom aus 75 Vol.-% H₂ und 25 Vol.-% N₂ durchgeführt. Zu 50 Nl / h des Gasstroms wurden 0,05 1 Zulauf aus Pyrrolidin/Wasser je Liter Katalysator und Stunde über einen Verdampfer eindosiert. Nach dem Reaktor wurden die Reaktionsprodukte über einen Intensivkühler mit nachgeschalteter Kühlfalle auskondensiert und gaschromatographisch analysiert.

Die Versuchsergebnisse sind in der nachstehenden Tabelle zusammengefasst.

**Tabelle**

| **H₂O** **[Gew.-%]** | **Pyrrol** **[GC-%]** | **Pyrrolidin** **[GC-%]** | **N-Butyl -Pyrrol** **[GC-%]** | **Butylamin** **[GC-%]** | **Butyronitril** **[GC-%]** | **Butyronitril/Pyrrol** **[GC-%]** |
|---|---|---|---|---|---|---|
| 5 | 82 | 4 | 4,2 | 1,4 | 0,13 | 0,15 |
| 10 | 83 | 6 | 2,7 | 1,8 | 0,09 | 0,10 |
| 15 | 79 | 11 | 1,3 | 2,5 | 0,05 | 0,06 |
| -* | 78 | 8 | 1,8 | 3,0 | 0,30 | 0,38 |

| | | | | | | |
|---|---|---|---|---|---|---|
| * Vergleichsbeispiel | | | | | | |

Wie der Tabelle zu entnehmen ist, führen bereits 5 Gew.-% Wasser im Einsatzstrom zu einer erheblichen Reduktion der Butyronitril-Bildung gegenüber dem Vergleichsbeispiel. Bei einem Wassergehalt von 15 Gew.-% beträgt die Butyronitril-Konzentration nur noch knapp ein Sechstel derjenigen, die beim Arbeiten in Abwesenheit von Wasser erhalten wird.

## Patentansprüche

1. Verfahren zum Herstellen von Pyrrolen der allgemeinen Formel (I) 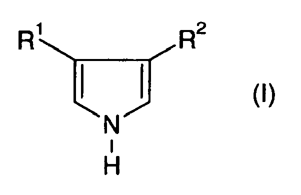 durch Dehydrieren von Pyrrolidinen der allgemeinen Formel (II) 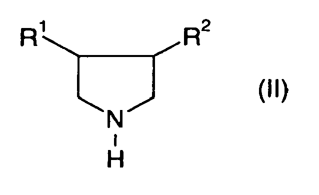 worin
R¹ und R² unabhängig voneinander Wasserstoff oder einen aliphatischen Rest mit 1 bis 6 C-Atomen bedeuten,
in Gegenwart eines geträgerten Edelmetallkatalysators, **dadurch gekennzeichnet, dass** man die Dehydrierung in Gegenwart von 1 bis 50 Gew.-% Wasser, bezogen auf das Pyrrolidin und Wasser, durchführt, wobei Wasser bereits dem Einsatzstrom der Dehydrierung beigemischt ist.

2. Verfahren nach Anspruch 1, **dadurch gekennzeichnet, dass** man die Dehydrierung in der Flüssigphase oder in der Gasphase durchführt.

3. Verfahren nach Anspruch 1, **dadurch gekennzeichnet, dass** man die Dehydrierung in der Gasphase in Gegenwart von Wasserstoff in einer Menge von 1 bis 100 mol je mol Pyrrolidin durchführt.

4. Verfahren nach einem der Ansprüche 1 bis 3, **dadurch gekennzeichnet, dass** man die Dehydrierung bei Temperaturen im Bereich von 150 bis 400°C und Drücken von 0,01 bis 50 bar durchführt.

5. Verfahren nach einem der Ansprüche 1 bis 4, **dadurch gekennzeichnet, dass** der Edelmetallkatalysator 30 bis 100 Gew.-%
a) Palladium auf einem Oxid einer Seltenen Erde oder einem Oxid eines Elements der Gruppe 4 des Periodensystems oder
b) eines Platin/Palladium-Gemischs auf Aluminiumoxid, einem Oxid einer Seltenen Erde oder einem Oxid eines Elements der Gruppe 4,
und 0 bis 70 Gew.-% Alkali- oder Erdalkalimetalloxid enthält.

6. Verfahren nach Anspruch 5, **dadurch gekennzeichnet, dass** man als Oxid einer Seltenen Erde ein Oxid des Cers, Praseodyms, Neodyms, Samariums, Europiums, Terbiums, Ytterbiums, Thoriums oder Protaktiniums einsetzt.

7. Verfahren nach Anspruch 5 oder 6, **dadurch gekennzeichnet, dass** man als Oxid eines Elements der Gruppe 4 ein Oxid des Titans oder Zirkoniums einsetzt.

8. Verfahren nach einem der Ansprüche 5 bis 7, **dadurch gekennzeichnet, dass** man als Alkali- oder Erdalkalimetalloxid ein Oxid des Natriums, Kaliums, Magnesiums, Calciums oder Bariums einsetzt.

## Claims

1. A process for the preparation of pyrroles of the formula (I) 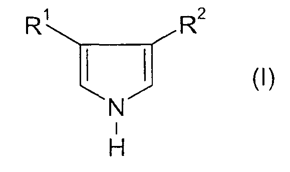 by dehydrogenating pyrrolidines of the formula (II) 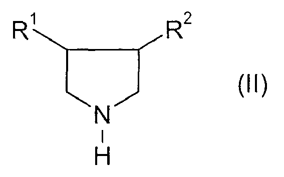 where
R¹ and R², independently of one another, are hydrogen or an aliphatic radical of 1 to 6 carbon atoms,
in the presence of a supported noble metal catalyst, wherein the dehydrogenation is carried out in the presence of from 1 to 50% by weight, based on the pyrrolidine and water, of water, where water has already been mixed with the feedstock stream of the dehydrogenation.

2. The process according to claim 1, wherein the dehydrogenation is carried out in the liquid phase or in the gas phase.

3. A process as claimed in claim 1, wherein the dehydrogenation is carried out in the gas phase in the presence of hydrogen in an amount of from 1 to 100 mol per mol of pyrrolidine.

4. A process as claimed in any of claims 1 to 3, wherein the dehydrogenation is carried out at from 150 to 400°C and from 0.01 to 50 bar.

5. A process as claimed in any of claims 1 to 4, wherein the noble metal catalyst comprises from 30 to 100% by weight of
a) palladium on an oxide of a rare earth metal or an oxide of an element of group 4 of the Periodic Table of the Elements or
b) a platinum/palladium mixture on alumina, an oxide of a rare earth metal or an oxide of an element of group 4,
and from 0 to 70% by weight of alkali metal or alkaline earth metal oxide.

6. A process as claimed in claim 5, wherein an oxide of cerium, of praseodymium, of neodymium, of samarium, of europium, of terbium, of ytterbium, of thorium or of protactinium is used as the oxide of a rare earth metal.

7. A process as claimed in claim 5 or 6, wherein an oxide of titanium or of zirconium is used as the oxide of an element of group 4.

8. A process as claimed in any of claims 5 to 7, wherein an oxide of sodium, of potassium, of magnesium, of calcium or of barium is used as the alkali metal or alkaline earth metal oxide.

## Revendications

1. Procédé de fabrication de pyrroles de formule générale (I) 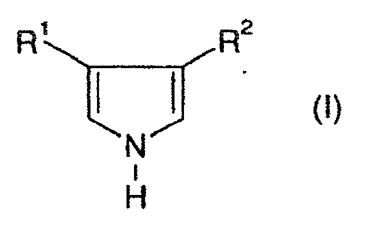 par déshydrogénation de pyrrolidines de formule générale (II) 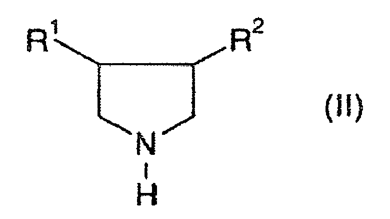 dans lesquelles
R¹ et R² représentent indépendamment l'un de l'autre l'hydrogène ou un radical aliphatique ayant 1 à 6 atomes C,
en présence d'un catalyseur supporté à base de métal noble, **caractérisé en ce que** la déshydrogénation est réalisée en présence de 1 à 50 % en poids d'eau, par rapport à la pyrrolidine et l'eau, l'eau étant déjà mélangée au courant d'alimentation de la déshydrogénation.

2. Procédé selon la revendication 1, **caractérisé en ce que** la déshydrogénation est réalisée dans la phase liquide ou dans la phase gazeuse.

3. Procédé selon la revendication 1, **caractérisé en ce que** la déshydrogénation est réalisée dans la phase gazeuse en présence d'hydrogène en une quantité de 1 à 100 moles par mole de pyrrolidine.

4. Procédé selon l'une quelconque des revendications 1 à 3, **caractérisé en ce que** la déshydrogénation est réalisée à des températures de 150 à 400 °C et des pressions de 0,01 à 50 bars.

5. Procédé selon l'une quelconque des revendications 1 à 4, **caractérisé en ce que** le catalyseur à base de métal noble contient 30 à 100 % en poids
a) de palladium sur un oxyde d'une terre rare ou un oxyde d'un élément du groupe 4 du tableau périodique ou
b) d'un mélange platine/palladium sur de l'oxyde d'aluminium, un oxyde d'une terre rare ou un oxyde d'un élément du groupe 4 du tableau périodique
et 0 à 70 % en poids d'oxyde de métal alcalin ou alcalino-terreux.

6. Procédé selon la revendication 5, **caractérisé en ce qu'**un oxyde de cérium, praséodyme, néodyme, samarium, europium, terbium, ytterbium, thorium ou protactinium est utilisé en tant qu'oxyde d'une terre rare.

7. Procédé selon la revendication 5 ou 6, **caractérisé en ce qu'**un oxyde de titane ou de zirconium est utilisé en tant qu'oxyde d'un élément du groupe 4.

8. Procédé selon l'une quelconque des revendications 5 à 7, **caractérisé en ce qu'**un oxyde de sodium, potassium, magnésium, calcium ou baryum est utilisé en tant qu'oxyde de métal alcalin ou alcalino-terreux.
